# FASCICULE DE BREVET EUROPEEN

(11) **EP 3 009 107 B1**
(45) Date de publication et mention de la délivrance du brevet: **05.04.2017**
(21) Numéro de dépôt: 15189276.7
(22) Date de dépôt: 12.10.2015
(51) Int. Cl.: A61F 2/46, A61F 2/42, A61F 2/34, A61F 2/00, A61F 2/30

(54) **ENSEMBLE COMPRENANT UN IMPLANT EN FORME DE CUPULE DE DIMENSIONS RÉDUITES ET PROCÉDÉ DE CONSTITUTION DE CET ENSEMBLE**
SET, DAS EIN PFANNENFÖRMIGES IMPLANTAT MIT REDUZIERTEN ABMESSUNGEN UMFASST, UND VERFAHREN ZUM ERSTELLEN DIESES SETS
ASSEMBLY COMPRISING A COMPACT CUP-SHAPED IMPLANT AND METHOD FOR FORMING SAID ASSEMBLY

(30) Priorité: 16.10.2014 FR 1459963
(43) Date de publication de la demande: 20.04.2016
(73) Titulaire: Groupe Lepine, 69730 Genay (FR)
(72) Inventeur: APARD, Thomas, 14000 Caen (FR); BARBARY, Stéphane, 54000 Nancy (FR); BOVET, Jean-Louis, 33320 Eysines (FR); DEWITTE, Gérard, 26200 Chateauneuf s/Isère (FR); KINNEN, Louis, 3040 Huldenberg (BE); TEISSIER, Jacques, 34170 Castelnau le Lez (FR); DRAI, Elsa, 69100 Villeurbanne (FR)
(74) Mandataire: Jeannet, Olivier

(56) Documents cités:
- EP-A1- 2 789 315
- FR-A1- 2 755 005
- FR-A1- 2 797 180
- US-A- 5 326 366

## Description

La présente invention concerne un ensemble comprenant un implant en forme de cupule de dimensions réduites et un préhenseur de cet implant. Elle concerne également un procédé de constitution de cet ensemble.

Ledit implant est une cupule de prothèse d'articulation de dimensions réduites, en particulier pour une articulation de la main ou du pied, et est notamment la cupule d'une prothèse trapézo-métacarpienne destinée à être ancrée dans l'os du trapèze.

Cet implant présente en outre une paroi continue formant intérieurement une surface d'articulation lisse et continue, et fait donc partie d'une prothèse dite "à double mobilité". Dans une telle prothèse, la cupule reçoit un noyau de glissement ayant une capacité de mouvements articulaires par rapport à elle.

Une prothèse trapézo-métacarpienne comprend généralement une tige médullaire destinée à être ancrée dans le premier métacarpien, une tête d'articulation prothétique reliée à cette tige médullaire, une cupule destinée à être ancrée au trapèze et un noyau de glissement destiné à être reçu dans la cupule, ce noyau formant une cavité articulaire dans laquelle est destinée à prendre place ladite tête d'articulation.

La solidité de l'ancrage de la cupule au trapèze est une condition généralement critique de la pérennité d'une prothèse trapézo-métacarpienne. Les dimensions réduites d'une telle cupule rendent ledit ancrage relativement difficile à réaliser. La manipulation de cette cupule est également difficile, pour la même raison. De plus, la dimension réduite de l'os du trapèze fait qu'avec les instruments actuels de préhension et d'impaction d'une telle cupule, il existe le risque que l'impaction de la cupule soit réalisée de façon relativement imprécise, c'est-à-dire non exactement dans le pré-trou aménagé dans cet os pour recevoir cette cupule. Il peut en résulter un endommagement de l'os, peu favorable à la solidité et à la pérennité de l'ancrage de la cupule.

La présente invention vise à remédier à ces inconvénients essentiels.

La publication de demande de brevet N° FR 2 797 180 A1 décrit un ensemble comprenant un implant en forme de cupule, ayant une paroi continue qui forme intérieurement une surface d'articulation lisse, et un préhenseur de cet implant ; ledit préhenseur comprend :
- une tête de préhension/impaction de l'implant, incluant une partie d'engagement destinée à être engagée dans la cavité délimitée par l'implant et à venir, dans une position d'engagement dans cette cavité, à proximité immédiate de la paroi de l'implant ; cette partie d'engagement comporte, sur l'ensemble de son pourtour, un joint d'étanchéité venant normalement, dans cette même position d'engagement, en application étanche contre ladite paroi ; et
- un manche de manipulation/impaction.

Le préambule de la revendication annexée est basé sur ce document.

Selon l'invention,
- l'implant est destiné à une articulation de la main ou du pied, et est donc de dimensions réduites ;
- le joint d'étanchéité est apte, lorsque l'ensemble est placé en dépression, à légèrement reculer dans le sens radial afin de laisser s'échapper l'air situé entre l'implant et la partie d'engagement ;
- la tête de préhension/impaction inclut une partie proximale de forme triangulaire, formant trois faces latérales, qui est dimensionnée de façon à ce que ces faces latérales soient inscrites, lorsque la tête de préhension/impaction est assemblée à l'implant, à l'intérieur du bord de cet implant délimitant l'ouverture de la cavité que délimite l'implant, cette partie proximale délimitant en outre une cavité d'assemblage permettant l'assemblage de la tête au manche de manipulation/impaction ; et
- ce manche de manipulation/impaction comprend un embout destiné à être inséré et coincé dans ladite cavité d'assemblage de manière à assembler ce manche à la tête de préhension/impaction.

Le procédé de constitution dudit ensemble comprend les étapes consistant à :
a) utiliser un implant et un préhenseur tels que précités ;
b) insérer ladite partie d'engagement dans la cavité de l'implant, jusqu'à ladite position d'engagement ;
d) réaliser une dépression d'air autour de l'implant et de la tête de préhension/impaction, de manière à faire s'échapper l'air situé entre l'implant et la partie d'engagement ; et
f) ramener ledit ensemble à la pression atmosphérique, de façon à réaliser l'assemblage de l'implant et de la tête de préhension/impaction au moyen de la dépression d'air crées entre cet implant et cette tête.

Ladite dépression d'air permet de créer une liaison entre l'implant et la tête de préhension/impaction, de sorte que cette tête, et le manche destiné à lui être associé, permettent de saisir et de manipuler l'implant facilement.

Lors de l'impaction de l'implant, le fait que ladite partie proximale de la tête ait une forme triangulaire et qu'elle soit dimensionnée de façon à être inscrite à l'intérieur dudit bord de cet implant, permet au praticien de parfaitement visualiser l'emplacement osseux dans lequel l'implant est destiné à être mis en place. Cette parfaite visualisation permet de prévenir efficacement tout risque d'impaction défectueuse de l'implant.

Une fois l'impaction réalisée, cette même forme triangulaire et ces mêmes dimensions de ladite partie proximale de la tête permettent que la tête puisse facilement être inclinée par rapport à l'implant afin de décoller légèrement un secteur du joint vis-à-vis de la paroi de l'implant, faisant ainsi entrer de l'air entre l'implant et ladite tête et éliminant de cette façon la dépression assurant la liaison de l'implant et de la tête. En effet, les faces latérales que présente ladite partie proximale de la tête forment des dégagements qui éliminent tout risque de venue de la tête au contact du bord de l'implant lors de cette inclinaison.

L'invention permet par conséquent de rendre possible l'utilisation d'une telle technique d'assemblage par le vide pour un implant de dimensions réduites, et permet de faciliter grandement la manipulation et l'impaction d'un tel implant.

De préférence, ledit ensemble comprend un élément de maintien appliqué étroitement autour de l'implant et de la tête de préhension/impaction de manière à maintenir cette tête dans une position coaxiale à l'implant.

Ce maintien élimine le risque d'inclinaison accidentelle de la tête de préhension/impaction par rapport à l'implant, susceptible de supprimer la liaison réalisée entre cette tête et l'implant.

De préférence, ledit élément de maintien est un emballage comprenant un corps de réception de l'implant et un couvercle conformé pour épouser ladite partie proximale de la tête de préhension/impaction, apte à être fixé sur ce corps.

Un parfait maintien de cet implant et de la tête de préhension/impaction est ainsi obtenu.

Ladite partie proximale est avantageusement tronquée au niveau des trois pointes que lui confère sa forme triangulaire, de telle sorte que non seulement lesdites faces latérales mais l'ensemble de cette partie proximale, donc y compris les facettes d'extrémité formées par lesdites pointes tronquées, soient inscrits à l'intérieur dudit bord de l'implant.

Cette disposition permet de réduire encore l'encombrement de cette partie proximale et donc d'améliorer encore la visibilité de la zone d'impaction au moment de cette impaction.

L'invention sera bien comprise, et d'autres caractéristiques et avantages de celle-ci apparaîtront, en référence au dessin schématique annexé ; ce dessin représente, à titre d'exemple non limitatif, une forme de réalisation préférée de l'ensemble implant-préhenseur/impacteur concerné.
La figure 1 est une vue en perspective éclatée de l'implant en forme de cupule ainsi que de la tête de préhension/impaction et d'une partie du manche que comprend le préhenseur/impacteur ;
la figure 2 est une vue de ces mêmes éléments de côté, en coupe passant par l'axe de l'implant, dans une position de préhension/impaction de l'implant ;
la figure 3 est une vue en perspective d'un corps d'emballage contenant l'implant et la tête de préhension/impaction ;
la figure 4 est une vue de ces éléments similaire à la figure 3, lorsque ledit manche est assemblé à ladite tête de préhension/impaction ; et
la figure 5 est une vue schématique, en perspective, d'un os du trapèze, en cours d'impaction de l'implant sur cet os.

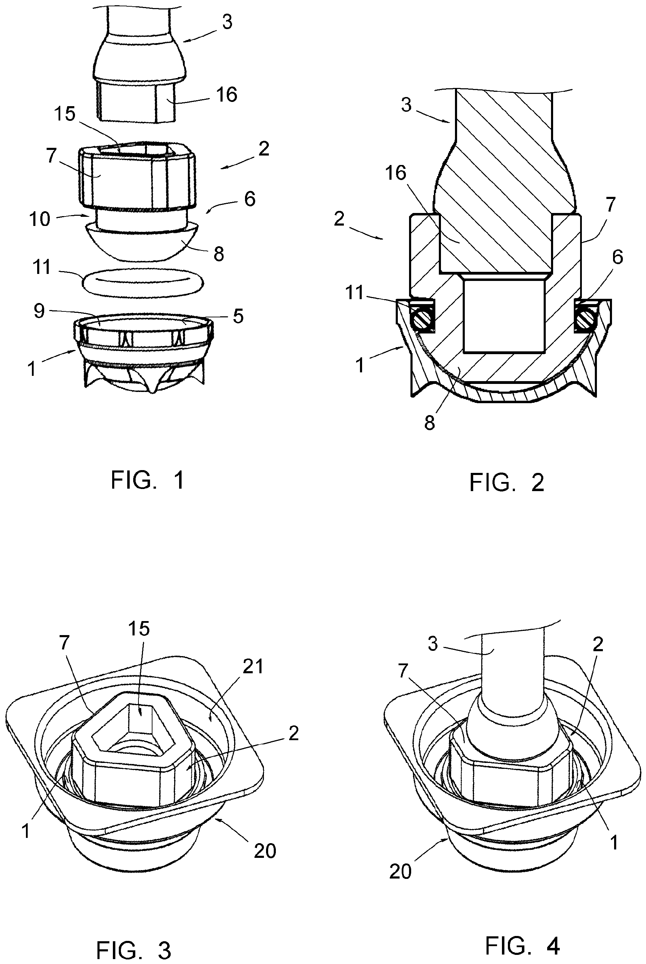

Les figures 1 et 2 représentent un implant 1 en forme de cupule et un préhenseur/impacteur de cet implant, incluant une tête 2 de préhension/impaction de l'implant 1 et un manche 3 de manipulation de l'ensemble tête 2 / implant 1.

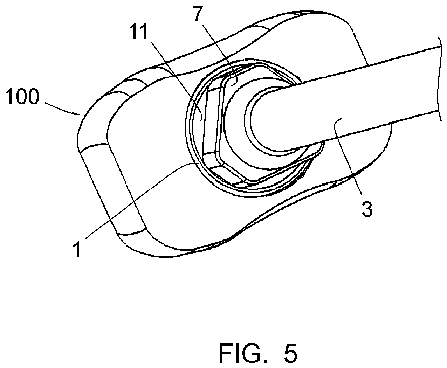

L'implant 1 est une cupule de prothèse d'articulation de dimensions réduites, faisant en particulier partie d'une prothèse d'articulation d'un doigt de la main ou du pied. Il s'agit notamment de la cupule d'une prothèse trapézo-métacarpienne destinée à être ancrée dans l'os 100 du trapèze, comme visible sur la figure 5.

L'implant 1 présente une paroi continue formant intérieurement une surface d'articulation 5 lisse et continue. Il fait donc partie d'une prothèse du type dit "à double mobilité", dans laquelle la cupule reçoit un noyau de glissement ayant une capacité de mouvements articulaires par rapport à elle.

Pour le reste, l'implant 1 est de type connu ; sa forme extérieure est notamment, dans l'exemple représenté, telle que décrite dans la publication de demande de brevet N° FR 2 883 723.

La tête de préhension/impaction 2 inclut une partie d'engagement 6 et une partie proximale 7.

La partie d'engagement 6 présente une portion 8 destinée à venir à proximité immédiate de la paroi de l'implant 1 lorsque cette partie d'engagement 6 est engagée dans la cavité 9 délimitée par l'implant 1, comme visible sur la figure 2. Elle comprend également, sur l'ensemble de son pourtour, une gorge 10 dans laquelle est placé un joint d'étanchéité 11, ce joint 11 venant normalement, dans cette même position d'engagement, en application étanche contre la paroi de l'implant 1 mais étant apte, lorsque l'ensemble implant 1 / tête 2 est placé en dépression, à légèrement reculer dans le sens radial afin de laisser s'échapper l'air situé entre l'implant 1 et ladite portion 8 de la partie d'engagement 6.

La partie proximale 7 a une forme triangulaire à pointes tronquées. Comme particulièrement visible sur les figures 2, 3 ou 5, elle est dimensionnée de façon à ce que les faces latérales qu'elle forme (tant les trois faces que lui confère sa forme triangulaire que les trois facettes intermédiaires formées par lesdites pointes tronquées) soient inscrites, lorsque la tête 2 est assemblée à l'implant 1, à l'intérieur du bord de cet implant délimitant l'ouverture de la cavité 9 que délimite l'implant.

La partie proximale 7 délimite en outre, en son centre, une cavité 15 ayant intérieurement la même forme qu'elle, et ayant une section allant en se réduisant très légèrement depuis l'ouverture de cette cavité vers le fond de celle-ci.

Le manche 3 comprend quant à lui un embout 16 destiné à être inséré et coincé dans la cavité 15 de manière à assembler ce manche 3 à la tête de préhension/impaction 2 de façon fiable mais avec possibilité de séparation manuelle.

Les figures 3 et 4 montrent le corps 20 d'un emballage contenant l'implant 1 et la tête de préhension/impaction 2 relié à lui par la dépression d'air précitée. Cet emballage comprend, outre le corps 20, un couvercle (non représenté), présentant une partie centrale saillante, apte à être engagée dans l'ouverture 21 du corps 20 et comprenant une empreinte apte à épouser la forme de la partie proximale 7. L'emballage forme ainsi un élément de maintien appliqué étroitement autour de l'implant 1 et de la tête 2, permettant de maintenir cette tête dans une position coaxiale à l'implant et donc d'éliminer le risque de suppression intempestive de la liaison par dépression d'air entre cet implant 1 et cette tête.

En pratique, après retrait dudit couvercle (cf. figure 3), l'ensemble tête 2 / implant 1 est saisi au moyen du manche 3, par simple insertion de l'embout 16 dans la cavité 15 (cf. figure 4), puis le préhenseur constitué par le manche 3 et la tête 2 est utilisé pour impacter l'implant 1 dans un trou de réception préparé dans l'os 100 (cf. figure 5). Comme cela est visible sur la figure 5, lors de cette impaction, la partie proximale 7 de la tête 2, du fait de sa forme triangulaire et de ses dimensions précitées, permet au praticien de parfaitement visualiser l'emplacement osseux dans lequel l'implant 1 est destiné à être impacté. Cette parfaite visualisation permet de prévenir efficacement tout risque d'impaction défectueuse de l'implant 1.

Une fois l'impaction réalisée, le manche 3, et donc la tête 2, peut être facilement incliné par rapport à l'implant 1 afin de décoller légèrement un secteur du joint 11 vis-à-vis de la paroi de l'implant 1, faisant ainsi entrer de l'air entre l'implant et la portion 8 de la tête 2, et éliminant de cette façon la dépression assurant la liaison de l'implant et de la tête. Lors de cette inclinaison, les faces latérales que présente la partie proximale 7 forment des dégagements qui éliminent tout risque de venue de la tête 2 au contact du bord de l'implant 1.

Comme cela apparaît de ce qui précède, l'invention a pour avantage déterminant de rendre possible l'utilisation d'une technique d'assemblage par le vide d'un préhenseur et d'un implant en forme de cupule ayant des dimensions réduites. L'ensemble implant-préhenseur qu'elle fournit permet de faciliter grandement la manipulation et l'impaction d'un tel implant.

L'invention a été décrite ci-dessus en référence à une forme de réalisation donnée à titre d'exemple. Il va de soi qu'elle n'est pas limitée à cette forme de réalisation mais qu'elle s'étend à toutes les formes de réalisation couvertes par les revendications ci-annexées.

## Revendications

1. Ensemble comprenant un implant (1) en forme de cupule, ayant une paroi continue qui forme intérieurement une surface d'articulation (5) lisse, et un préhenseur de cet implant (1) ; ledit préhenseur comprend :
- une tête (2) de préhension/impaction de l'implant (1), incluant une partie d'engagement (6) destinée à être engagée dans la cavité (9) délimitée par l'implant (1) et à venir, dans une position d'engagement dans cette cavité, à proximité immédiate de la paroi de l'implant (1) ; cette partie d'engagement (6) comporte, sur l'ensemble de son pourtour, un joint d'étanchéité (11) venant normalement, dans cette même position d'engagement, en application étanche contre ladite paroi ; et
- un manche (3) de manipulation/impaction ;
**caractérisé en ce que** :
- l'implant (1) est destiné à une articulation de la main ou du pied, et est donc de dimensions réduites ;
- le joint d'étanchéité (11) est apte, lorsque l'ensemble est placé en dépression, à légèrement reculer dans le sens radial afin de laisser s'échapper l'air situé entre l'implant (1) et la partie d'engagement (6) ;
- la tête (2) de préhension/impaction inclut une partie proximale (7) de forme triangulaire, formant trois faces latérales, qui est dimensionnée de façon à ce que ces faces latérales soient inscrites, lorsque la tête de préhension/impaction (2) est assemblée à l'implant (1), à l'intérieur du bord de cet implant (1) délimitant l'ouverture de la cavité (9) que délimite l'implant (1), cette partie proximale (7) délimitant en outre une cavité d'assemblage (15) permettant l'assemblage de la tête (2) au manche (3) de manipulation/impaction ; et
- ce manche (3) de manipulation/impaction, comprenant un embout (16) destiné à être inséré et coincé dans ladite cavité d'assemblage (15) de manière à assembler ce manche (3) à la tête de préhension/impaction (2).

2. Ensemble selon la revendication 1, **caractérisé en ce qu'**il comprend un élément de maintien appliqué étroitement autour de l'implant (1) et de la tête de préhension/impaction (2) de manière à maintenir cette tête dans une position coaxiale à l'implant (1).

3. Ensemble selon la revendication 2, **caractérisé en ce que** ledit élément de maintien est un emballage comprenant un corps (20) de réception de l'implant (1) et un couvercle apte à être fixé sur ce corps, conformé pour épouser ladite partie proximale (7) de la tête de préhension/impaction (2).

4. Ensemble selon l'une des revendications 1 à 3, **caractérisé en ce que** ladite partie proximale (7) est tronquée au niveau des trois pointes que lui confère sa forme triangulaire, de telle sorte que non seulement lesdites faces latérales mais l'ensemble de cette partie proximale (7), donc y compris les facettes d'extrémité formées par lesdites pointes tronquées, soient inscrits à l'intérieur dudit bord de l'implant (1).

5. Procédé de constitution de l'ensemble selon l'une des revendications 1 à 4, **caractérisé en ce qu'**il comprend les étapes consistant à :
a) utiliser un implant (1) et un préhenseur tels que précités ;
b) insérer ladite partie d'engagement (6) dans la cavité (9) de l'implant (1), jusqu'à ladite position d'engagement ;
d) réaliser une dépression d'air autour de l'implant (1) et de la tête de préhension/impaction (2), de manière à faire s'échapper l'air situé entre l'implant (1) et la partie d'engagement (6) ; et
f) ramener ledit ensemble à la pression atmosphérique, de façon à réaliser l'assemblage de l'implant (1) et de la tête de préhension/impaction (2) au moyen de la dépression d'air crées entre cet implant (1) et cette tête.

## Patentansprüche

1. Anordnung, umfassend ein pfannenförmiges Implantat (1) mit einer kontinuierlichen Wand, die innen eine glatte Gelenkoberfläche (5) bildet, sowie einen Greifer für dieses Implantat (1), wobei der Greifer umfasst:
- einen Kopf (2) zum Greifen/Beaufschlagen des Implantats (1), umfassend einen Eingriffsbereich (6), der dazu ausgelegt ist, in Eingriff in dem Hohlraum (9) zu sein, der durch das Implantat (1) begrenzt ist, und in einer Eingriffsposition in diesem Hohlraum in die unmittelbare Nähe der Wand des Implantats (1) zu gelangen, wobei dieser Eingriffsbereich (6) über seinem gesamten Umfang eine Dichtung (11) umfasst, die normalerweise in dieser gleichen Eingriffsposition in dichte Anlage gegen die Wand gelangt, und
- einen Stiel (3) zur Handhabung/Beaufschlagung;
**dadurch gekennzeichnet, dass**;
- das Implantat (1) für ein Gelenk der Hand oder des Fußes bestimmt ist und somit reduzierte Abmessungen aufweist;
- die Dichtung (11) dazu ausgelegt ist, sich dann, wenn die Anordnung unter Unterdruck gesetzt wird, in der radialen Richtung leicht zurückzuziehen, um die Luft entweichen zu lassen, die sich zwischen dem Implantat (1) und dem Eingriffsbereich (6) befindet;
- der Greif-/Beaufschlagungskopf (2) einen proximalen Bereich (7) mit dreieckiger Form umfasst, der drei laterale Flächen bildet, und der derart dimensioniert ist, dass diese drei lateralen Flächen, wenn der Greif-/Beaufschlagungskopf (2) an dem Implantat (1) montiert ist, im Inneren des Rands dieses Implantats (1) eingeschrieben sind, der die Öffnung des Hohlraums (9) begrenzt, den das Implantat (1) begrenzt, wobei dieser proximale Bereich (7) ferner einen Zusammenfügungshohlraum (15) begrenzt, der die Zusammenfügung des Kopf (2) mit dem Handhabungs-/Beaufschlagungsstiel (3) erlaubt; und
- dieser Handhabungs-/Beaufschlagungsstiel (3) einen Ansatz (16) umfasst, der dazu ausgelegt ist, in den Zusammenfügungshohlraum (15) derart eingesetzt und gepresst zu werden, dass dieser Stiel (3) mit dem Greif-/Beaufschlagungskopf (2) zusammengefügt ist.

2. Anordnung nach Anspruch 1, **dadurch gekennzeichnet, dass** sie ein Halteelement umfasst, das eng um das Implantat und den Greif-/Beaufschlagungskopf (2) gedrückt ist, derart, dass dieser Kopf in einer Position koaxial zum Implantat (1) gehalten wird.

3. Anordnung nach Anspruch 2, **dadurch gekennzeichnet, dass** das Halteelement eine Verpackung ist, umfassend einen Körper (20) zur Aufnahme des Implantats (1) und einen Deckel, der dazu ausgelegt ist, an diesem Körper befestigt zu sein, mit einer Gestalt passend zum proximalen Bereich (7) des Greif-/ Beaufschlagungskopfs (2).

4. Anordnung nach einem der Ansprüche 1 bis 3, **dadurch gekennzeichnet, dass** der proximale Bereich (7) im Bereich der drei Spitzen, die ihm seine dreieckige Form verleiht, verjüngt ist, derart, dass nicht nur die lateralen Flächen, sondern die Gesamtheit dieses proximalen Bereichs (7) einschließlich der Endfacetten, die durch die verjüngten Spitzen gebildet sind, im Inneren des Rands des Implantats (1) eingeschrieben ist.

5. Verfahren zur Herstellung der Anordnung nach einem der Ansprüche 1 bis 4, **dadurch gekennzeichnet, dass** es die folgenden Schritte umfasst:
a) Verwenden eines Implantats (1) und eines Greifers wie vorstehend genannt;
b) Einsetzen des Eingriffsbereichs (6) in den Hohlraum (9) des Implantats (1) bis zur Eingriffsposition;
d) Realisieren eines Luftunterdrucks um das Implantat (1) und den Greif-/ Beaufschlagungskopf (2) herum derart, dass die Luft entweicht, die sich zwischen dem Implantat (1) und dem Eingriffsbereich (6) befindet; und
f) Rückführen der Anordnung auf Atmosphärendruck derart, dass die Zusammenfügung des Implantats (1) und des Greif-/Beaufschlagungskopfs (2) mit Hilfe des Luftunterdrucks realisiert wird, der zwischen diesem Implantat (1) und diesem Kopf erzeugt wird.

## Claims

1. Assembly comprising a cup-shaped implant (1) having a continuous wall which internally forms a smooth articulation surface (5) and a gripper for this implant (1); said gripper comprises:
- a head (2) for gripping / impaction of the implant (1), including an engagement portion (6) intended to be engaged in the cavity (9) delimited by the implant (1) and to come, in a position of engagement in this cavity, in the immediate vicinity of the wall of the implant (1); this engagement portion (6) comprises, over the whole of its periphery, a sealing gasket (11) coming normally, in said engagement position, in a sealed engagement against said wall; and
- a manipulation / impaction handle (3);
**characterized in that**:
- the implant (1) is intended for a joint of the hand or the foot, and is therefore of reduced dimensions;
- the sealing gasket (11) is capable, when the assembly is placed under reduced pressure, to slightly retract in the radial direction in order to allow air between the implant (1) and the engagement portion (6) to escape;
- the gripping / impaction head (2) includes a triangular proximal portion (7) forming three lateral surfaces, which is dimensioned such that these lateral surfaces are inscribed, when the gripping / impaction head (2) is joined to the implant (1), within the edge of this implant (1) delimiting the opening of the cavity (9) delineated by the implant (1), this proximalportion (7) further delineating a mounting cavity (15) enabling assembly of the head (2) to the manipulation / impaction handle (3); and
- this manipulation / impaction handle (3) comprising a tip (16) intended to be inserted and wedged in said mounting cavity (15) so as to assemble said handle (3) with the gripping / impaction head (2).

2. Assembly according to claim 1, **characterized in that** it comprises a holding element applied tightly around the implant (1) and the gripping / impaction head (2) so as to maintain this head in a position coaxial to the implant (1).

3. Assembly as claimed in claim 2, **characterized in that** said holding element is a package comprising a body (20) for receiving the implant (1) and a cover adapted to be fixed to the body, shaped to conform to said proximal portion (7) of the gripping / impaction head (2).

4. Assembly as claimed in claim 1, wherein said proximal portion is truncated at the level of the three points formed by its triangular shape so that not only said lateral surfaces but the whole proximal portion (7), thus including the end facets formed by said truncated points, are inscribed inside said edge of the implant (1).

5. Method of constituting the assembly according to one of claims 1-4, **characterized in that** it comprises the steps consisting in:
a) using an implant (1) and a gripper as mentioned above;
b) inserting said engaging portion (6) in the cavity (9) of the implant (1), to said engaging position;
d) producing an air depression around the implant (1) and the gripping / impaction head (2) so as to cause the air between the implant (1) and the engagement portion (6) to escape; and
f) returning said assembly to atmospheric pressure, so as to achieve assembly of the implant (1) and of the gripping / impaction head (2) by means of the air depression created between this implant (1) and this head.
